# EUROPEAN PATENT APPLICATION

(11) **EP 4 289 575 A1**
(43) Date of publication of application: **13.12.2023**
(21) Application number: 22749656.9
(22) Date of filing: 31.01.2022
(51) Int. Cl.: B26B 19/42, A61B 5/00, A61B 5/107, B26B 19/28

(54) **BEAUTIFICATION MACHINE FOR CUTTING HAIR, AND BEAUTIFICATION SYSTEM**

(30) Priority: 04.02.2021 JP 2021016414
(71) Applicant: Panasonic Intellectual Property Management Co., Ltd., Osaka-shi, Osaka 540-6207 (JP)
(72) Inventor: NAKAGAWA Takehiro, Kadoma-shi Osaka 571-0057 (JP); SUGIURA Akiho, Kadoma-shi Osaka 571-0057 (JP); KASUGAI Hideki, Kadoma-shi Osaka 571-0057 (JP); OKUDA Ichiro, Kadoma-shi Osaka 571-0057 (JP)
(74) Representative: Grünecker Patent- und Rechtsanwälte PartG mbB
(86) International application number: PCT/JP2022/003570
(87) International publication number: WO 2022/168784

(57) **Abstract**

The present disclosure provides a beautification machine for cutting hair and a beautification system capable of giving a person being treated with a more appropriate skin care treatment. Beautification machine for cutting hair (10) according to the present disclosure includes main body (20) including treatment unit (230) capable of cutting hair. Main body (20) includes skin information acquisition unit (30) that acquires skin information, and threshold acquisition unit (60) that acquires a threshold for determining a state of skin based on the skin information acquired by skin information acquisition unit (30). Main body (20) includes treatment setting unit (80) that sets an operation state of treatment unit (230) based on the skin information acquired by skin information acquisition unit (30) and the threshold acquired by threshold acquisition unit (60).

## Description

### TECHNICAL FIELD

The present disclosure relates to a beautification machine for cutting hair and a beautification system.

### BACKGROUND ART

Conventionally, as disclosed in PTL 1, an electronic treatment device including ion introduction means, pulse supply means, a capacitive semiconductor sensor, and diagnosis means for diagnosing a state of skin based on a capacitance value signal or the like has been proposed.

In PTL 1, the electronic treatment device further includes treatment setting means for setting execution contents of each treatment based on a diagnosis result of the skin with the diagnosis means.

It is possible to perform an effective treatment without waste by setting a type and execution content of the treatment in accordance with the state of the skin of a person being treated.

### Citation List

### Patent Literature

PTL 1: Unexamined Japanese Patent Publication No. 2004-141259

### SUMMARY OF THE INVENTION

### Technical problem

An object of the present disclosure is to provide a beautification machine for cutting hair and a beautification system capable of giving a more appropriate skin care treatment to a person being treated.

### Solution to problem

A beautification machine for cutting hair according to the present disclosure includes a main body including a treatment unit capable of cutting hair. In addition, the main body includes a skin information acquisition unit that acquires skin information, and a threshold acquisition unit that acquires a threshold for determining a state of skin based on the skin information acquired by the skin information acquisition unit. The main body includes a treatment setting unit that sets an operation state of the treatment unit based on the skin information acquired by the skin information acquisition unit and the threshold acquired by the threshold acquisition unit.

A beautification system according to the present disclosure includes the beautification machine for cutting hair. In addition, the beautification system includes at least one means of an information display unit capable of displaying the skin information, a threshold transmission unit capable of transmitting data of the threshold acquired by the threshold acquisition unit, and a skin information transmission unit capable of transmitting data of the skin information acquired by the skin information acquisition unit. Advantageous effect of invention

According to the present disclosure, a more appropriate skin care treatment can be given to a person being treated only by using a beautification machine for a hair cutting purpose. As a result, when hair is cut by using the beautification machine for cutting hair, a more appropriate skin care treatment can be given to the person being treated.

### BRIEF DESCRIPTION OF THE DRAWINGS

Fig. 1 is a perspective view schematically illustrating a beautification machine for cutting hair according to a first exemplary embodiment.
Fig. 2 is a perspective view schematically illustrating an inner blade included in the beautification machine for cutting hair according to the first exemplary embodiment.
Fig. 3 is a diagram schematically illustrating a use state of the beautification machine for cutting hair according to the first exemplary embodiment.
Fig. 4 is a diagram for explaining an attachment position of a sensor according to the first exemplary embodiment.
Fig. 5 is a block diagram illustrating an example of a main body according to the first exemplary embodiment.
Fig. 6 is a flowchart illustrating an example of operation state setting processing of a treatment unit according to the first exemplary embodiment.
Fig. 7 is a block diagram illustrating an example of a main body according to a modification of the first exemplary embodiment.
Fig. 8 is a block diagram illustrating an example of a basic function controller according to the modification of the first exemplary embodiment.
Fig. 9 is a diagram schematically illustrating a beautification system according to a second exemplary embodiment.
Fig. 10 is a diagram schematically illustrating a beautification system according to a first modification of the second exemplary embodiment.
Fig. 11 is a diagram schematically illustrating a beautification system according to a second modification of the second exemplary embodiment.

### DESCRIPTION OF EMBODIMENT

### (Findings and like underlying present disclosure)

At a time when the inventors conceived of the present disclosure, an electronic treatment device described in PTL 1 was known.

In addition, there is also known a beautification machine for a hair cutting purpose (that is, beautification machine for cutting hair) including a treatment unit capable of cutting hair, such as an electric razor and a hair clipper. When the hair of the skin of a person being treated is cut by using such a beautification machine for cutting hair, the beautification machine for cutting hair may influence the skin.

Thus, it is preferable that the influence of the beautification machine for cutting hair on the skin can be reduced at the time of using the beautification machine for cutting hair. However, but since a human skin property has a large individual difference, it has been difficult to uniformly set a coping method.

Thus, in a case where the beautification machine for cutting hair influences the skin, the electronic treatment device disclosed in PTL 1 is used to care for the skin.

However, when skin care is performed by using the electronic treatment device as in PTL 1, there is a problem that it is necessary to secure a time for treatment and it takes time and effort to perform a work. In addition, there is also a problem that it is difficult for a general person being treated to accurately grasp a skin state at a current point in time. Furthermore, since time restraints and troublesome works increase, there is also a problem that even though the beautification machine for cutting hair influences the skin, appropriate care cannot be performed depending on the person being treated.

Under such circumstances, the inventors have obtained an idea of giving a more appropriate skin care treatment to the person being treated when the beautification machine for a hair cutting purpose is used.

With such an idea, the inventors have come to constitute the subject matter of the present disclosure.

The present disclosure provides a beautification machine for cutting hair and a beautification system capable of giving a more appropriate skin care treatment to a person being treated.

Hereinafter, exemplary embodiments will be described in detail with reference to the drawings. However, unnecessarily detailed description may not be described. For example, a detailed description of already well-known matters or a redundant description of substantially the same configuration may be omitted.

Note that, the accompanying drawings and the following description are only presented to help those skilled in the art fully understand the present disclosure, and are not intended to limit the subject matters described in the scope of claims.

In addition, in the following exemplary embodiments, an electric razor that mainly cuts a beard which is a body hair (that is, one type of hair) of a person or an animal is illustrated as an example of the beautification machine for cutting hair.

Here, in the following exemplary embodiments, a longitudinal direction of a grip part of the electric razor is defined as an up-down direction, a direction in which a head part protrudes from the grip part (that is, longitudinal direction of the head part) is defined as a width direction, and a direction orthogonal to the up-down direction and the width direction is defined as a front-rear direction.

In addition, in the present exemplary embodiment, a side of the grip part where the head part is arranged is defined as an upper side in the up-down direction, and a side of the grip part where a switch part is provided is defined as a front side in the front-rear direction.

### (First exemplary embodiment)

Electric razor 10 (that is, one type of beautification machine for cutting hair) according to the present exemplary embodiment includes main body 20 including treatment unit 230 capable of cutting hair. In the present exemplary embodiment, treatment unit 230 includes blade part 240 for cutting hair.

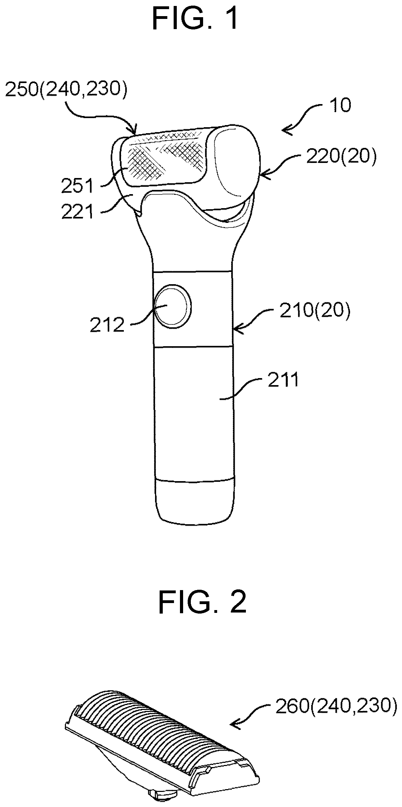

In addition, as illustrated in Fig. 1, main body 20 includes grip part 210 to be gripped by hand and head part 220 supported by grip part 210. Blade part 240 is attached to head part 220. Preferably, blade part 240 is detachably attached to head part 220. Note that, head part 220 does not have to be provided in the electric razor.

Grip part 210 is made of, for example, synthetic resin or the like, and includes grip housing 211 having a space therein, and various components such as a substrate for supplying electric power for driving electric razor 10 are incorporated in grip housing 211.

In addition, push switch part 212 that operates (that is, turns on and off a power supply to) electric razor 10 is formed in grip housing 211. Note that, although push switch part 212 is illustrated as an example of the switch part in the present exemplary embodiment, a slide switch or another switch may be used as long as the switch turns on and off the power supply.

In the present exemplary embodiment, switch part 212 is formed at a front surface of grip part 210, that is, at a front (that is, forward) surface of electric razor 10. Note that, the front surface of electric razor 10 is a surface on a side where the electric razor faces person being treated U in a state where grip part 210 of electric razor 10 is gripped by person being treated U in normal use.

As illustrated in Fig. 1, head part 220 includes head housing 221 attached to grip part 210, and a drive mechanism (not illustrated) is accommodated in head housing 221. Conventionally and publicly known examples of the drive mechanism that can be used include an oscillatory linear actuator and a drive mechanism that includes a rotary motor and a conversion mechanism that converts a rotation motion to a linear reciprocation motion.

On the other hand, as illustrated in Figs. 1 and 2, blade part 240 includes outer blade 250 that is exposed to an upper side from head part 220, and inner blade 260 that is disposed on an inner side of outer blade 250 (that is, lower side of outer blade 250) and moves relatively to outer blade 250.

A large number of blade holes (not illustrated) are formed in outer blade 250, and outer blade 250 is arranged such that the large number of blade holes are exposed to the upper side from head part 220. A portion of outer blade 250 exposed to the upper side from head part 220 is blade surface (that is, shaving surface) 251 that comes into contact with skin S of person being treated U. As described above, in the present exemplary embodiment, blade part 240 has blade surface 251 capable of coming into contact with skin S when hair is cut.

On the other hand, inner blade 260 is attached to the above-described drive mechanism (not illustrated), and when the drive mechanism is driven, inner blade 260 reciprocates in the width direction (that is, in the longitudinal direction).

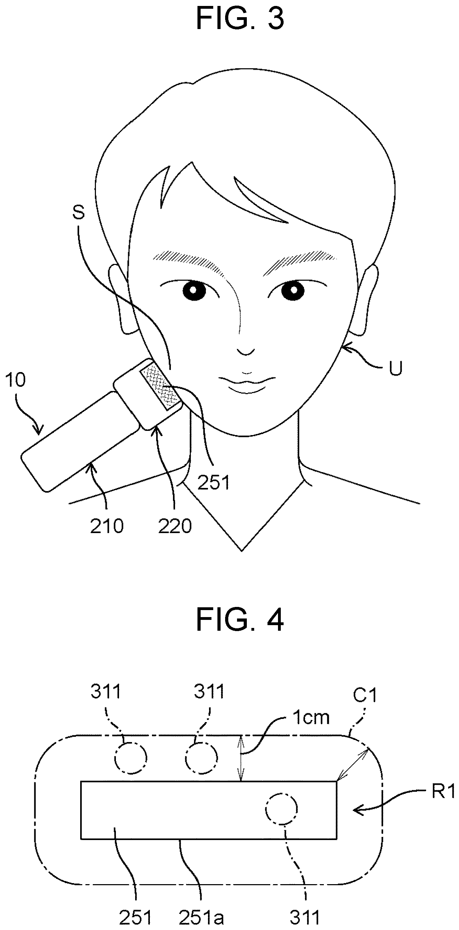

Such blade part 240 is provided on an upper portion of head part 220, and thus, as illustrated in Fig. 3, person being treated U can shave hair (for example, a beard or the like) of skin S by causing skin S pressed against blade surface 251 to protrude to an inner side from the blade holes.

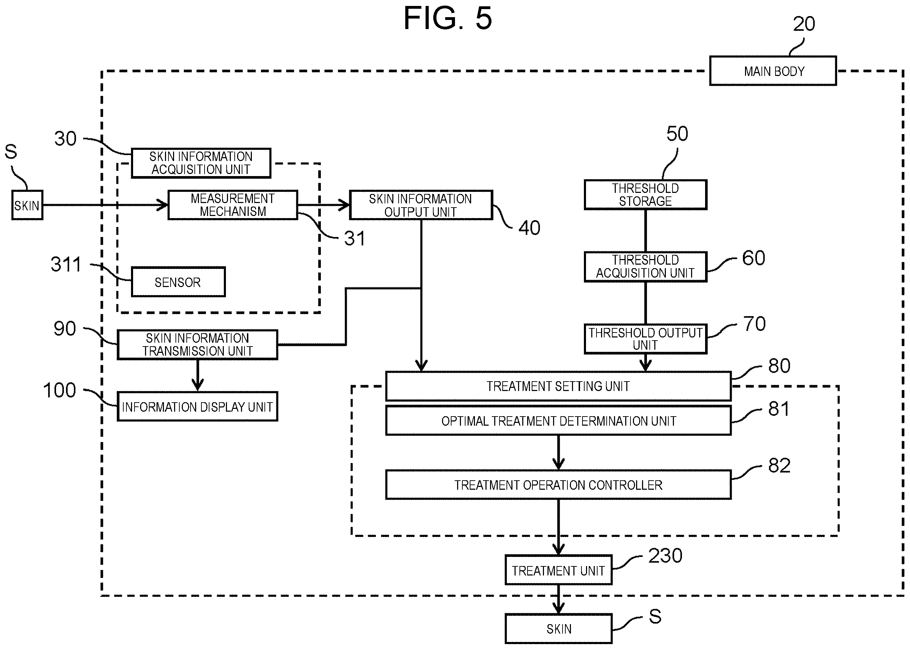

As illustrated in Fig. 5, in the present exemplary embodiment, main body 20 includes sensor 311. Preferably, sensor 311 can measure a state of skin S (that is, the same surface as the shaving surface) having hair to be cut by blade part 240. That is, electric razor 10 preferably has a function as a sensor shaver including sensor 311 capable of measuring the same surface as the shaving surface.

For example, an electrode sensor, a non-contact sensor, an infrared sensor, an optical sensor, an image sensor, an imaging sensor, a moisture sensor, a microscope, or the like can be used as such sensor 311. At this time, electric razor 10 may include two or more sensors, or may include two or more types of sensors.

In addition, as illustrated in Fig. 4, sensor 311 is preferably arranged in region R1 in which a position 1 cm away from outer shell 251a of blade surface 251 is outer shell C1.

By doing this, since sensor 311 is installed near blade part 240 that is a basic function, the state of skin S immediately after hair cutting (that is, immediately after the treatment by blade part 240) and immediately before cutting can be measured.

Note that, in a case where sensor 311 installed near blade part 240 is configured to measure the state of skin S immediately after hair cutting, immediately before sensor 311 measures the state of skin S, it is possible to cut a beard present in a portion of skin S to be measured by blade part 240. When the beard present in the portion of skin S to be measured are cut by blade part 240, sensor 311 is prevented from malfunctioning due to the beard present in the portion of skin S to be measured. As described above, in a case where sensor 311 installed near blade part 240 is configured to measure the state of skin S immediately after hair cutting, in the use of electric razor 10 (that is, one type of beautification machine for cutting hair), blade part 240 has not only a function of simply cutting a beard present on skin S, but also a function as an automatic beard cutting unit that cuts an unnecessary beard so as not to cause sensor 311 to malfunction.

In addition, in the present exemplary embodiment, as will be described later, a more appropriate skin care treatment can be given to person being treated U only by using electric razor 10. Thus, when sensor 311 is arranged in region R1, more appropriate skin care can be selected and performed based on the state of skin S immediately after hair cutting (that is, immediately after the treatment by blade part 240). As a result, more appropriate skin care can be selected and performed without depending on the skill of person being treated U. In addition, a hair cutting treatment can be performed with a more appropriate pressing pressure based on the state of skin S immediately before hair cutting.

In the present exemplary embodiment, as illustrated in Fig. 5, main body 20 includes skin information acquisition unit 30 that acquires skin information, and threshold acquisition unit 60 that acquires a threshold (that is, shaver operation threshold data) for determining the state of skin S based on skin information acquired by skin information acquisition unit 30. In addition, main body 20 includes treatment setting unit 80 that sets an operation state of treatment unit 230 based on the skin information acquired by skin information acquisition unit 30 and the threshold acquired by threshold acquisition unit 60.

Furthermore, in the present exemplary embodiment, skin information acquisition unit 30 includes measurement mechanism 31 (that is, skin state detection unit) capable of measuring the state of skin S, and measurement mechanism 31 includes sensor 311 capable of measuring the state of skin S.

At this time, measurement mechanism 31 (that is, skin state detection unit) can be configured to detect the state of skin S by using at least one or more indexes indicating skin characteristics. Examples of the indexes indicating the skin characteristics include moisture amount, oil amount, texture, wrinkle, spot, dullness, acne, shine, pore state, disturbance of horny layer, scale, and the like.

With such a configuration of main body 20, it is possible to perform a treatment for more appropriate skin care for skin S of person being treated U in accordance with the state of skin S acquired by skin information acquisition unit 30.

In addition, in the present exemplary embodiment, main body 20 includes skin information output unit 40 that outputs the skin information acquired by skin information acquisition unit 30 to treatment setting unit 80. Accordingly, the skin information acquired by skin information acquisition unit 30 is sent to treatment setting unit 80 via skin information output unit 40.

In addition, in the present exemplary embodiment, main body 20 includes threshold storage 50 that stores a threshold, and threshold acquisition unit 60 acquires the threshold stored in threshold storage 50. Main body 20 includes threshold output unit 70 that outputs the threshold acquired by threshold acquisition unit 60 to treatment setting unit 80. Accordingly, in the present exemplary embodiment, data of the threshold stored in advance in threshold storage 50 is acquired by threshold acquisition unit 60, and the threshold acquired by threshold acquisition unit 60 is sent to treatment setting unit 80 via threshold output unit 70. In the present exemplary embodiment, setting value A and setting value B having a larger value than setting value A are stored as the data of the threshold in advance in threshold storage 50.

In addition, in the present exemplary embodiment, main body 20 includes skin information transmission unit 90 that transmits information to information display unit 100 capable of displaying the skin information. With such a configuration, person being treated U can know his or her own information from information display unit 100 every day. In the present exemplary embodiment, information display unit 100 is provided on main body 20, and information display unit 100 can be, for example, a display provided on main body 20 of electric razor 10. The skin information acquired by skin information acquisition unit 30 is sent to skin information output unit 40 and is sent to information display unit 100 via skin information transmission unit 90. By doing this, person being treated U or the like can check the skin information displayed on information display unit 100. Information display unit 100 can display update notification of the skin information, the threshold, and the like.

In addition, treatment setting unit 80 sets the operation state of treatment unit 230 based on the skin information acquired by skin information acquisition unit 30 and the threshold acquired by threshold acquisition unit 60.

In the present exemplary embodiment, treatment setting unit 80 includes optimal treatment determination unit 81 for determining an optimal treatment method, and treatment operation controller 82 for operating treatment unit 230 by the optimal treatment method (that is, treatment control value) determined by optimal treatment determination unit 81.

In the present exemplary embodiment, optimal treatment determination unit 81 determines an optimal treatment method (that is, sets the treatment control value) by comparing the skin information acquired by skin information acquisition unit 30 with the threshold acquired by threshold acquisition unit 60.

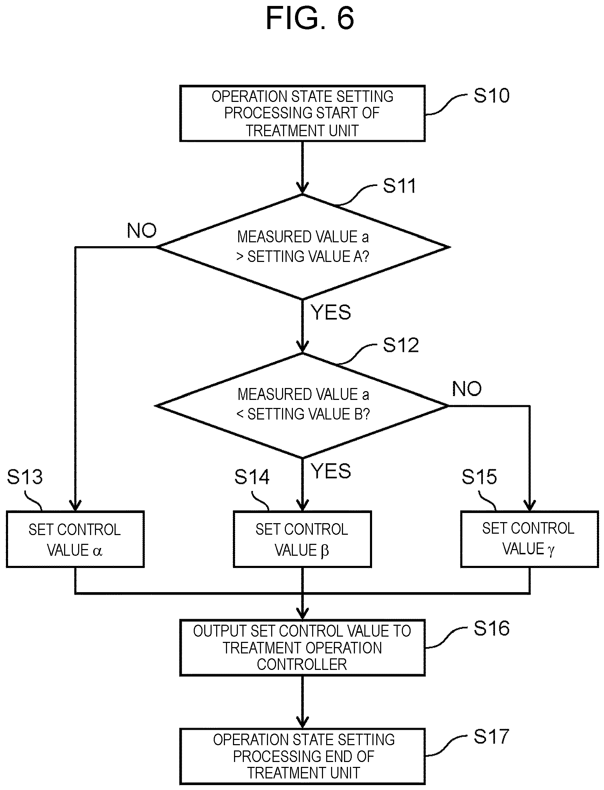

Specifically, the optimal treatment method is determined (that is, the treatment control value is set) by performing processing (that is, operation state setting processing of treatment unit 230) illustrated in Fig. 6.

In the operation state setting processing of treatment unit 230 illustrated in Fig. 6, first, in step S10, the operation state setting processing of treatment unit 230 is started. Subsequently, in step S 11, the skin information (that is, measured value a) sent from skin information output unit 40 is compared with setting value A sent from threshold output unit 70. Specifically, it is determined whether or not the skin information (that is, measured value a) sent from skin information output unit 40 is larger than setting value A sent from threshold output unit 70.

When it is determined as "YES" in step S11, the processing proceeds to step S12.

That is, in step S12, it is determined whether or not the skin information (that is, measured value a) sent from skin information output unit 40 is smaller than setting value B sent from threshold output unit 70.

Note that, in a case where "NO" is determined in step S11, the processing proceeds to step S13, and control value α is set.

In a case where it is determined as "YES" in step S12, the processing proceeds to step S14, and control value β is set.

On the other hand, in a case where it is determined as "NO" in step S12, the processing proceeds to step S15, and control value γ is set.

In step S16, the control value set in any one of steps S13 to S15 is output to treatment operation controller 82.

In step S17, the operation state setting processing of treatment unit 230 is terminated.

By doing this, treatment operation controller 82 operates treatment unit 230 by the optimal treatment method (that is, treatment control value) determined by optimal treatment determination unit 81.

In a case where treatment unit 230 includes a member for performing care for skin S by irradiating skin S with light, such as a flash lamp, control of changing at least one selected from the group consisting of a wavelength and intensity of light with which skin S is irradiated in accordance with the state of skin S can be mentioned as an example of such control. Note that, in a case where care for skin S is performed by stimulating cell activity by applying electrical stimulation, ultrasonic waves, or the like to skin S, for example, it is possible to perform control for changing the electrical stimulation applied to skin S, the intensity of the ultrasonic waves, a cycle, or the like in accordance with the state of skin S.

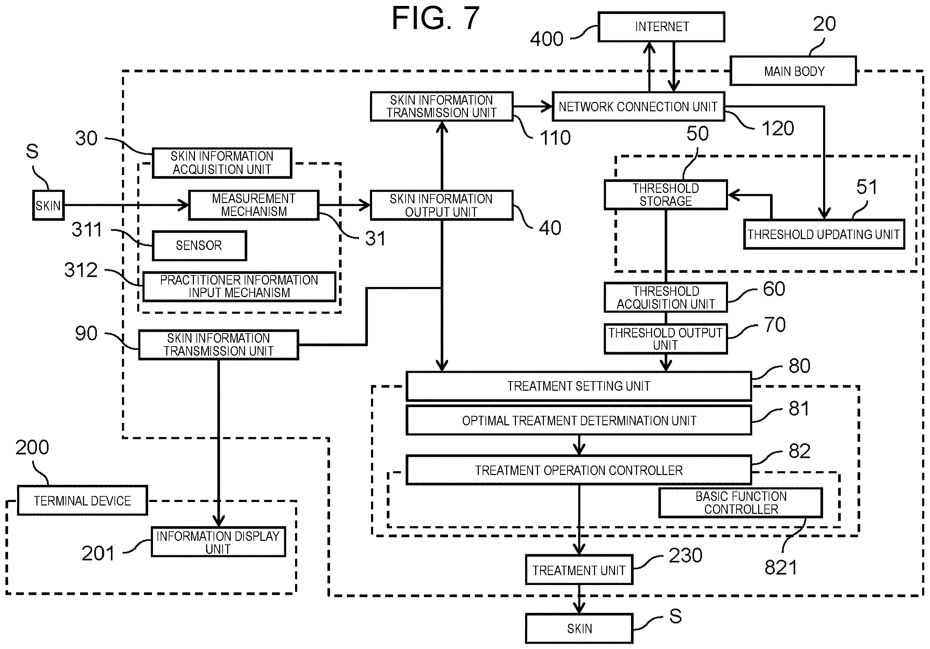

In addition, main body 20 may have a configuration illustrated in Fig. 7 illustrating a modification of the present exemplary embodiment.

Main body 20 illustrated in Fig. 7 includes skin information acquisition unit 30 that acquires the skin information, and threshold acquisition unit 60 that acquires the threshold (that is, shaver operation threshold data) for determining the state of skin S based on the skin information acquired by skin information acquisition unit 30. In addition, main body 20 illustrated in Fig. 7 includes treatment setting unit 80 that sets the operation state of treatment unit 230 based on the skin information acquired by skin information acquisition unit 30 and the threshold acquired by threshold acquisition unit 60.

Skin information acquisition unit 30 includes measurement mechanism 31 (that is, skin state detection unit) capable of measuring the state of skin S, and measurement mechanism 31 includes sensor 311 capable of measuring the state of skin S.

Here, in main body 20 illustrated in Fig. 7, skin information acquisition unit 30 includes practitioner information input mechanism 312 capable of inputting the state of skin S of person being treated U. By doing this, the skin information (for example, a skin trouble or the like that person being treated U wants to improve) that person being treated U is interested in to is input practitioner information input mechanism 312, it is possible to perform a more suitable treatment for a current state of skin S of person being treated U. Note that, examples of the skin information that person being treated U is interested in include various kinds of information such as dry skin S and deep wrinkles formed on skin S.

In addition, main body 20 illustrated in Fig. 7 includes skin information output unit 40 that outputs the skin information acquired by skin information acquisition unit 30 to treatment setting unit 80.

Furthermore, main body 20 illustrated in Fig. 7 includes threshold storage 50 that stores the threshold, and threshold output unit 70 that outputs the threshold acquired by threshold acquisition unit 60 to treatment setting unit 80.

Here, main body 20 illustrated in Fig. 7 include threshold updating unit 51 (that is, threshold external input unit) capable of updating the threshold stored in threshold storage 50 based on threshold information acquired from an outside of electric razor 10 (that is, one type of beautification machine for cutting hair).

Furthermore, main body 20 illustrated in Fig. 7 includes network connection unit 120 that can be connected to a network system such as Internet 400. Threshold updating unit 51 is connected to the network system via network connection unit 120, and threshold updating unit 51 can update the threshold stored in threshold storage 50 by acquiring latest threshold information.

As described above, in main body 20 illustrated in Fig. 7, threshold updating unit 51 is provided, and thus, the threshold for determining the optimal treatment method can be updated from the knowledge obtained by academia or the like, big data obtained by acquisition of biological information or the like to more reliable information.

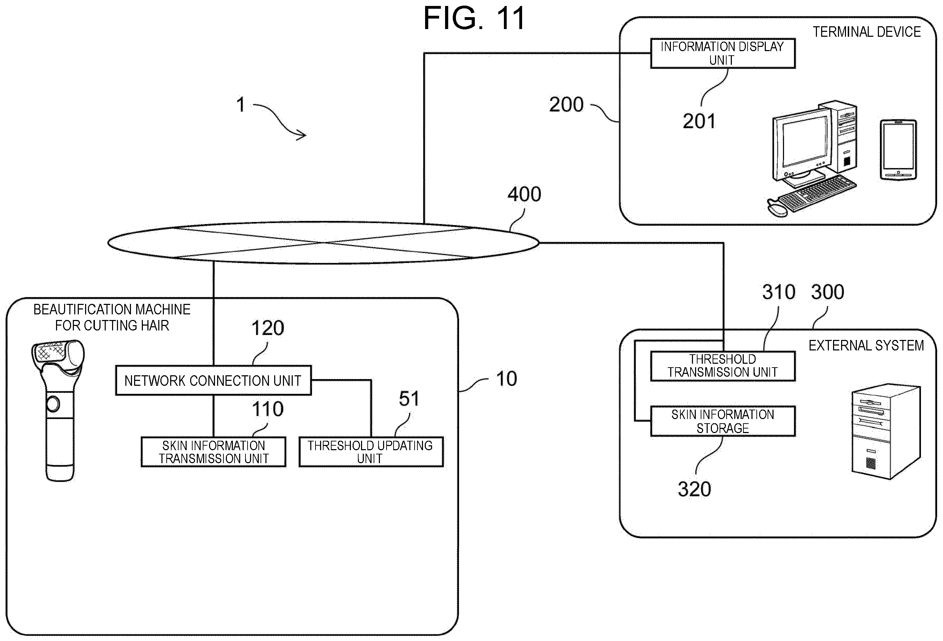

In addition, main body 20 illustrated in Fig. 7 includes skin information transmission unit 110 capable of transmitting the skin information acquired by skin information acquisition unit 30 to the outside (for example, external system 300 such as a server, and see Fig. 11). In main body 20 illustrated in Fig. 7, skin information transmission unit 110 is also connected to the network system via network connection unit 120. The data sent to skin information transmission unit 110 can be transmitted to the outside (for example, external system 300 such as a server) via the network system.

In addition, main body 20 illustrated in Fig. 7 includes skin information transmission unit 90 that transmits information to information display unit 201 capable of displaying the skin information. Here, in main body 20 illustrated in Fig. 7, information display unit 201 is provided in terminal device 200 such as a personal computer or a smartphone. Information display unit 201 can also be, for example, a display provided in terminal device 200. Information display unit 201 can display the update notification of the skin information, the threshold, and the like.

In addition, treatment setting unit 80 includes optimal treatment determination unit 81 for determining the optimal treatment method, and treatment operation controller 82 for operating treatment unit 230 by the optimal treatment method (that is, treatment control value) determined by optimal treatment determination unit 81.

Here, in main body 20 illustrated in Fig. 7, treatment setting unit 80 includes basic function controller 821 that controls the basic function of treatment unit 230. Specifically, treatment operation controller 82 includes basic function controller 821 that controls the basic function of treatment unit 230.

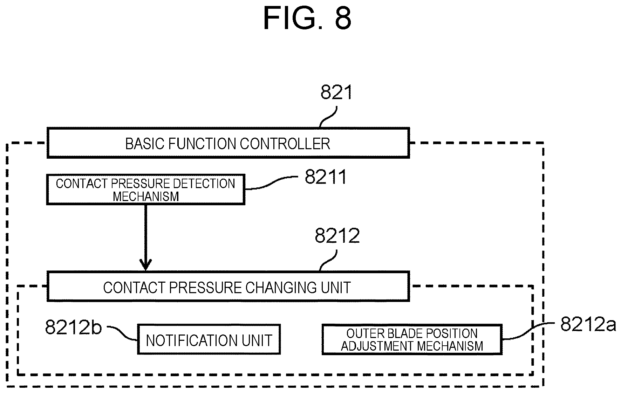

In addition, as illustrated in Fig. 8, basic function controller 821 includes contact pressure detection mechanism 8211 (for example, a skin contact pressure detection unit) that detects a contact pressure of outer blade 250 to skin S. Furthermore, basic function controller 821 further includes contact pressure changing unit 8212 (for example, a blade surface pressing reduction position adjustment unit) capable of changing the contact pressure based on the contact pressure detected by contact pressure detection mechanism 8211.

By doing this, the contact pressure of outer blade 250 to skin S in the use of electric razor 10 is set to appropriate pressure. Electric razor 10 can be used in a more suitable state for the treatment in treatment unit 230.

Specifically, contact pressure changing unit 8212 includes outer blade position adjustment mechanism 8212a (that is, outer blade position adjustment unit) that adjusts a position of outer blade 250.

By doing this, the pressing of blade surface 251 can be reduced by adjusting the position of outer blade 250 based on a skin state (for example, the contact pressure of outer blade 250 to skin S), and control can be performed such that the contact pressure becomes more appropriate in the use of electric razor 10. Movable control of outer blade 250 by outer blade position adjustment mechanism 8212a can be performed by using, for example, a solenoid. In addition, optimal treatment determination unit 81 may reduce the pressing based on the current skin state.

In addition, contact pressure changing unit 8212 may include notification unit 8212b (for example, a user pressing strong and weak force guiding unit) that notifies a contact state of outer blade 250 with skin S. Examples of a method for notifying the contact state of outer blade 250 with skin S include sound, vibration, and light.

By doing this, the skin state (for example, the contact pressure of outer blade 250 to skin S) can be notified to person being treated U, and in a case where the pressure of person being treated U is strong, the person being treated can be guided to weaken the pressure.

### (Second exemplary embodiment)

In the present exemplary embodiment, beautification system 1 including beautification machine for cutting hair 10 described in the first exemplary embodiment and the modification thereof will be described.

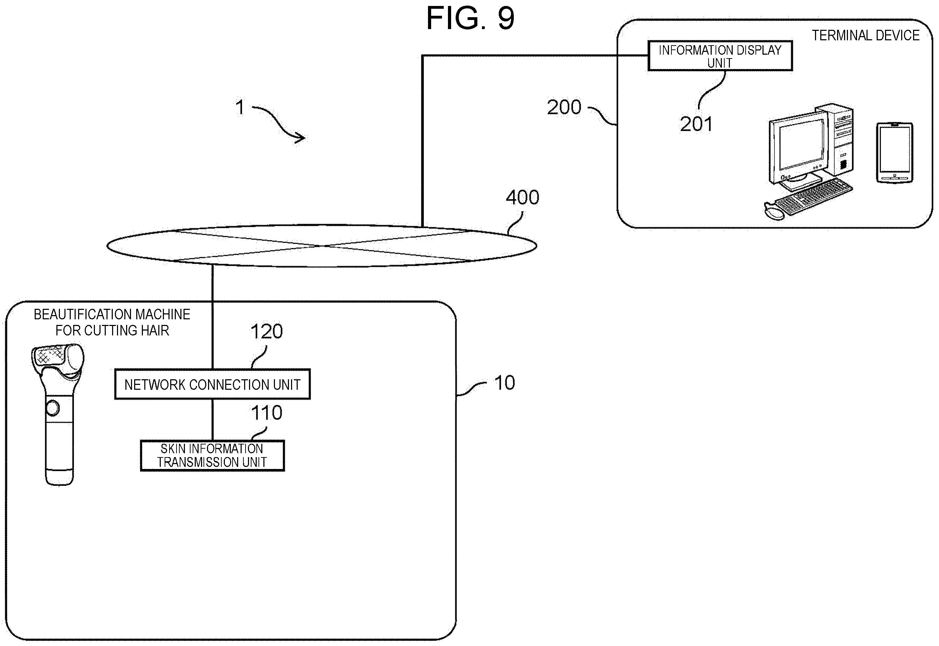

As illustrated in Fig. 9, beautification system 1 according to the present exemplary embodiment includes electric razor 10 (that is, one type of beautification machine for cutting hair) including skin information transmission unit 110, and information display unit 201 capable of displaying the skin information.

In the present exemplary embodiment, information display unit 201 capable of displaying the skin information is provided in terminal device 200 such as a personal computer or a smartphone.

In addition, electric razor 10 (that is, one type of beautification machine for cutting hair) includes network connection unit 120 that can be connected to a network system such as Internet 400. The data sent to skin information transmission unit 110 can be transmitted to information display unit 201 of terminal device 200 via the network system.

By doing this, the skin information (for example, a graph of a temporal change, an image indicating a current skin state, and the like) acquired by skin information acquisition unit 30 can be checked via information display unit 201 provided in terminal device 200 such as a personal computer or a smartphone.

As described above, beautification system 1 according to the present exemplary embodiment includes electric razor 10 (that is, one type of beautification machine for cutting hair) capable of outputting the measured value to the outside.

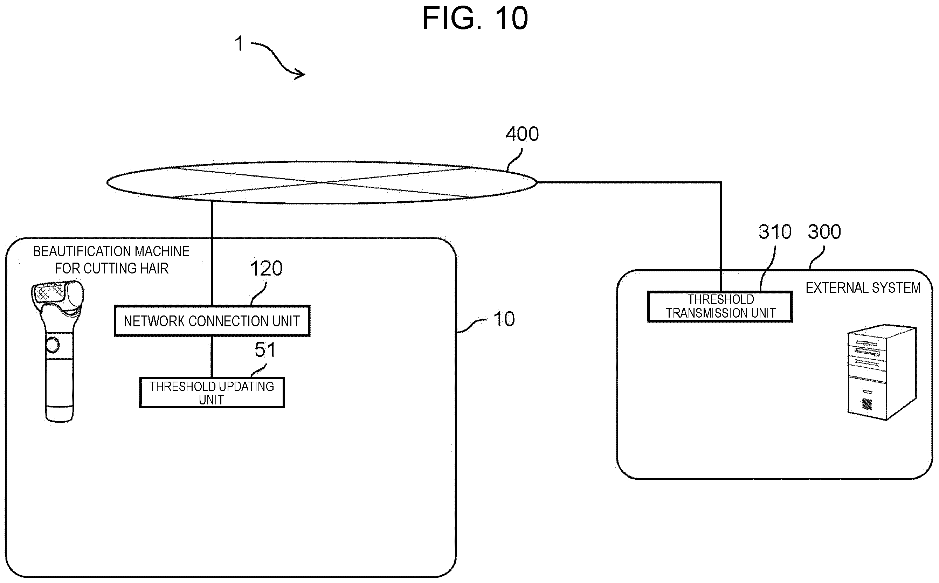

In addition, beautification system 1 can have a configuration illustrated in Fig. 10.

Beautification system 1 illustrated in Fig. 10 includes electric razor 10 (that is, one type of beautification machine for cutting hair) including threshold updating unit 51 (that is, threshold external input unit), and threshold transmission unit 310 capable of transmitting data of the threshold acquired by threshold acquisition unit 60.

In beautification system 1 illustrated in Fig. 10, threshold transmission unit 310 capable of transmitting the data of the threshold acquired by threshold acquisition unit 60 is provided in external system 300 such as a server.

In addition, electric razor 10 (that is, one type of beautification machine for cutting hair) includes network connection unit 120 that can be connected to a network system such as Internet 400. The data of the threshold sent from threshold transmission unit 310 can be transmitted to threshold updating unit 51 (that is, threshold external input unit) via the network system.

By doing this, threshold updating unit 51 (that is, threshold external input unit) updates the threshold stored in threshold storage 50 based on the data of the threshold sent from threshold transmission unit 310, and the updated threshold is acquired by threshold acquisition unit 60.

In addition, beautification system 1 can have a configuration illustrated in Fig. 11.

Beautification system 1 illustrated in Fig. 11 includes electric razor 10 (that is, one type of beautification machine for cutting hair) including skin information transmission unit 110 and threshold updating unit 51 (that is, threshold external input unit), and information display unit 201 capable of displaying the skin information. In addition, beautification system 1 illustrated in Fig. 11 includes threshold transmission unit 310 capable of transmitting the data of the threshold acquired by threshold acquisition unit 60, and skin information storage 320 capable of storing the data sent to skin information transmission unit 110.

In beautification system 1 illustrated in Fig. 11, information display unit 201 capable of displaying the skin information is provided in terminal device 200 such as a personal computer or a smartphone.

In addition, in beautification system 1 illustrated in Fig. 11, threshold transmission unit 310 capable of transmitting the data of the threshold acquired by threshold acquisition unit 60 and skin information storage 320 capable of storing the data sent to skin information transmission unit 110 are provided in external system 300 such as a server.

In addition, electric razor 10 (that is, one type of beautification machine for cutting hair) includes network connection unit 120 that can be connected to a network system such as Internet 400. The data sent to skin information transmission unit 110 can be transmitted to information display unit 201 of terminal device 200 via the network system.

By doing this, the skin information (for example, a graph of a temporal change, an image indicating a current skin state, and the like) acquired by skin information acquisition unit 30 can be checked via information display unit 201 provided in terminal device 200 such as a personal computer or a smartphone.

In addition, the data of the threshold sent from threshold transmission unit 310 can be transmitted to threshold updating unit 51 (that is, threshold external input unit) via the network system.

By doing this, threshold updating unit 51 (that is, threshold external input unit) updates the threshold stored in threshold storage 50 based on the data of the threshold sent from threshold transmission unit 310, and the updated threshold is acquired by threshold acquisition unit 60.

Furthermore, the data sent to skin information transmission unit 110 can be transmitted to skin information storage 320 of external system 300 via the network system.

By doing this, skin information data of person being treated U can be utilized for feedback to the person being treated or can be utilized as big data.

### [Actions and effects]

Hereinafter, a description will be given of a characteristic configuration of the beautification machine for cutting hair and the beautification system described in each of the above exemplary embodiments and the modification thereof, and an effect obtained by the characteristic configuration.
(1) Beautification machine for cutting hair 10 described in each of the above exemplary embodiments and the modification includes main body 20 that includes treatment unit 230 capable of cutting hair. In addition, main body 20 includes skin information acquisition unit 30 that acquires skin information, and threshold acquisition unit 60 that acquires a threshold for determining a state of skin S based on the skin information acquired by skin information acquisition unit 30. Furthermore, main body 20 includes treatment setting unit 80 that sets an operation state of treatment unit 230 based on the skin information acquired by skin information acquisition unit 30 and the threshold acquired by threshold acquisition unit 60.

In other words,
beautification machine for cutting hair 10 described in each of the exemplary embodiments and the modification thereof includes:
main body 20 that includes treatment unit 230 capable of cutting hair;
skin information acquisition unit 30 that acquires skin information; and
electric circuitry which, in operation,
   acquires skin information;
   acquires a threshold for determining a state of skin S based on the skin information; and
   sets an operation state of treatment unit 230 based on the skin information and the threshold.

Examples of the electrical and electronic circuit are an integrated circuit (that is, IC) and a large scale integrated circuit (that is, LSI).

By doing this, when hair is cut by using beautification machine for cutting hair 10, a more appropriate skin care treatment can be given to person being treated U. That is, it is possible to give a more appropriate skin care treatment to person being treated U only by using beautification machine for cutting hair 10.

Furthermore, there is also an advantage that more appropriate skin care can be selected and performed based on the acquired skin information even though person being treated U does not know the skin information and does not secure a new treatment time.

As described above, when beautification machine for cutting hair 10 described in each of the exemplary embodiments and the modification thereof is used, it is possible to perform the skin treatment suitable for the current state of person being treated U without providing an additional time, and care for skin stress caused by beautification machine for cutting hair 10.

(2) In addition, skin information acquisition unit 30 may include measurement mechanism 31 capable of measuring the state of skin S.

By doing this, more appropriate skin care can be selected and performed based on the state of skin S measured by measurement mechanism 31.

(3) In addition, measurement mechanism 31 may include sensor 311 capable of measuring the state of skin S.

By doing this, more appropriate skin care can be selected and performed based on the state of skin S measured by sensor 311.

(4) In addition, treatment unit 230 may include blade part 240 having blade surface 251 capable of coming into contact with skin S when hair is cut. Sensor 311 may be arranged in region R1 where a position 1 cm away from outer shell 251a of blade surface 251 is outer shell C1.

By doing this, sensor 311 is installed near blade part 240 that is the basic function, the state of skin S immediately after hair cutting (that is, immediately after the treatment by blade part 240) can be known. More appropriate skin care can be selected and performed based on the state of skin S immediately after hair cutting (that is, immediately after the treatment by blade part 240). Thus, it is possible to select and perform more appropriate skin care without depending on the skill of person being treated U.

(5) In addition, skin information acquisition unit 30 may include practitioner information input mechanism 312 capable of inputting the state of skin S of person being treated U.

By doing this, skin state data of interest to person being treated U can be input to practitioner information input mechanism 312. Then, it is possible to select and perform more appropriate skin care based on skin state data of interest of person being treated U input to practitioner information input mechanism 312.

(6) In addition, main body 20 may include skin information output unit 40 that outputs the skin information acquired by skin information acquisition unit 30 to treatment setting unit 80.

By doing this, the skin information acquired by skin information acquisition unit 30 can be output from skin information output unit 40 to treatment setting unit 80.

(7) In addition, main body 20 may include skin information transmission unit 110 capable of transmitting the skin information acquired by skin information acquisition unit 30 to an outside.

By doing this, the skin information acquired by skin information acquisition unit 30 can be transmitted from skin information transmission unit 110 to the outside.

(8) In addition, main body 20 may include threshold storage 50 that stores the threshold, and threshold acquisition unit 60 may acquire the threshold stored in threshold storage 50.

An example of threshold storage 50 is a nonvolatile memory.

By doing this, threshold acquisition unit 60 can acquire the threshold stored in threshold storage 50. The operation state of treatment unit 230 can be set by using the threshold stored in threshold storage 50.

(9) In addition, threshold storage 50 may include threshold updating unit 51 capable of updating the threshold stored in threshold storage 50 based on threshold information acquired from an outside of beautification machine for cutting hair 10.

By doing this, the threshold stored in threshold storage 50 can be updated to latest information, and more appropriate skin care can be selected and performed.

(10) In addition, main body 20 may include threshold output unit 70 that outputs the threshold acquired by threshold acquisition unit 60 to treatment setting unit 80.

By doing this, the threshold acquired by skin information acquisition unit 30 can be output from threshold output unit 70 to treatment setting unit 80.

(11) In addition, treatment setting unit 80 may include basic function controller 821 that controls a basic function of treatment unit 230.

By doing this, it is possible to control an operation state by blade part 240 that is the basic function of treatment unit 230.

(12) In addition, treatment unit 230 may include outer blade 250. Basic function controller 821 may include contact pressure detection mechanism 8211 that detects a contact pressure of outer blade 250 to skin S, and contact pressure changing unit 8212 capable of changing the contact pressure based on the contact pressure detected by contact pressure detection mechanism 8211.

By doing this, the contact pressure of outer blade 250 to skin S can be controlled to be more appropriate. As a result, when hair is cut by using beautification machine for cutting hair 10, the influence on skin S of person being treated U can be further reduced.

(13) In addition, contact pressure changing unit 8212 may include outer blade position adjustment mechanism 8212a that adjusts a position of outer blade 250.

By doing this, the position of outer blade 250 can be adjusted in order to reduce the pressing of blade surface 251 from skin state (for example, the contact pressure of outer blade 250 to skin S), and the contact pressure can be controlled to be more appropriate.

(14) In addition, contact pressure changing unit 8212 may include notification unit 8212b that gives a notification of a contact state of outer blade 250 with skin S.

By doing this, skin state (for example, the contact pressure of outer blade 250 to skin S) can be notified to person being treated U, and it is possible to guide person being treated U to weaken the pressure.

(15) In addition, main body 20 may include skin information transmission unit 90 that transmits information to information display units 100 and 201 capable of displaying the skin information.

By doing this, since the skin state of person being treated U, the update information of the threshold data, and the like can be displayed on information display units 100 and 201, usability of beautification machine for cutting hair 10 can be further improved.

(16) In addition, skin information transmission unit 90 may transmit information to information display unit 100 provided in main body 20.

Accordingly, various pieces of information can be checked only via beautification machine for cutting hair 10 without using other devices such as terminal device 200. That is, it is not necessary to prepare another device such as terminal device 200 in order to check the information. As a result, it is possible to save time and effort when the information is checked.

(17) In addition, skin information transmission unit 90 may transmit information to information display unit 201 provided in terminal device 200.

By doing this, since various pieces of information can be checked via terminal device 200, various pieces of information can be checked while beautification machine for cutting hair 10 is used.

(18) In addition, beautification system 1 includes beautification machine for cutting hair 10 described in any one of (1) to (17), and information display unit 201 capable of displaying the skin information.

As described above, in beautification system 1 including beautification machine for cutting hair 10 and information display unit 201, various pieces of information such as the state of skin S of person being treated U can be checked while beautification machine for cutting hair 10 is used.

(19) In addition, beautification system 1 may include beautification machine for cutting hair 10 described in any one of above (1) to (17) and threshold transmission unit 310 capable of transmitting data of the threshold acquired by threshold acquisition unit 60.

By doing this, since the data of the threshold transmitted from threshold transmission unit 310 can be acquired by threshold acquisition unit 60 included in beautification machine for cutting hair 10, the operation state of treatment unit 230 can be set by using the latest threshold data.

(20) In addition, beautification system 1 may include beautification machine for cutting hair 10 described in any one of above (1) to (17), and skin information transmission unit 130 capable of transmitting data of the skin information acquired by skin information acquisition unit 30.

By doing this, the skin information acquired by skin information acquisition unit 30 can be transmitted from skin information transmission unit 130 to the outside. Note that, the skin information transmitted to the outside can be used as information for proposing a more appropriate treatment (that is, obtaining more optimal threshold information) or can be used to accumulate the acquired data as a database and use the data for another business.

### [Others]

Contents of the beautification machine for cutting hair and the beautification system according to the present disclosure have been described above, but it is obvious to those skilled in the art that various modifications and improvements are possible without limitation to the descriptions.

For example, it is possible to provide a beautification machine for cutting hair in which the configurations described in the above exemplary embodiments and the modification thereof are appropriately combined.

In addition, in the exemplary embodiments and the modification thereof, although electric razor 10 is illustrated as an example of the beautification machine for cutting hair, the present disclosure is not limited thereto, and can be applied to hair clippers, trimmers, and the like.

In addition, in the exemplary embodiments and the modification thereof, threshold acquisition unit 60 acquires the threshold stored in threshold storage 50. However, it is not necessary to provide threshold storage 50, and threshold acquisition unit 60 may directly acquire the threshold data present outside such as skin information storage 320 of external system 300.

In addition, measurement mechanism 31 (that is, skin state detection unit) may include a contact pressure detection mechanism (for example, skin contact pressure detection unit).

In addition, the beautification machine for cutting hair may include a shaver operation determination unit that determines an operation of a shaver by comparing data regarding the skin state detected by the skin state detection unit to the shaver operation threshold data for determining the operation of the shaver.

In addition, the shaver operation determination unit may include a threshold external input unit capable of externally inputting the shaver operation threshold data.

In addition, the shaver operation determination unit may include an optimal treatment determination unit that determines an optimal treatment method.

In addition, the optimal treatment determination unit may learn by an artificial intelligence function (hereinafter, referred to as an "AI function").

In addition, specifications (for example, a shape, a size, a layout, and the like) of the treatment setting unit, the skin information acquisition unit, and other details can be changed as appropriate.

### INDUSTRIAL APPLICABILITY

As described above, since the beautification machine for cutting hair and the beautification system according to the present disclosure can give a more appropriate skin care treatment to a person being treated, the beautification machine for cutting hair and the beautification system can be used for various kinds of beautification machines for cutting hair and beautification systems including home use and business use.

### REFERENCE MARKS IN THE DRAWINGS

1 beautification system
10 electric razor (that is, one type of beautification machine for cutting hair)
20 main body
230 treatment unit
240 blade part
250 outer blade
251 blade surface
251a outer shell
30 information acquisition unit
31 measurement mechanism
311 sensor
312 practitioner information input mechanism
40 skin information output unit
50 threshold storage
51 threshold updating unit
60 threshold acquisition unit
70 threshold output unit
80 treatment setting unit
821 basic function controller
8211 contact pressure detection mechanism
8212 contact pressure changing unit
8212a outer blade position adjustment mechanism
8212b notification unit
90 skin information transmission unit
100 information display unit
110 skin information transmission unit
200 terminal device
201 information display unit
310 threshold transmission unit
C1 outer shell
R1 region
S skin
U person being treated

## Claims

1. A beautification machine for cutting hair, comprising:
a main body that comprises a treatment unit capable of cutting hair, wherein
the main body comprises:
a skin information acquisition unit that acquires skin information;
a threshold acquisition unit that acquires a threshold for determining a state of skin based on the skin information acquired by the skin information acquisition unit; and
a treatment setting unit that sets an operation state of the treatment unit based on the skin information acquired by the skin information acquisition unit and the threshold acquired by the threshold acquisition unit.

2. The beautification machine for cutting hair according to Claim 1, wherein
the skin information acquisition unit comprises a measurement mechanism capable of measuring the state of the skin.

3. The beautification machine for cutting hair according to Claim 2, wherein
the measurement mechanism comprises a sensor capable of measuring the state of the skin.

4. The beautification machine for cutting hair according to Claim 3, wherein
the treatment unit comprises a blade part having a blade surface capable of coming into contact with the skin of which hair is cut, and
the sensor is disposed in a region where a position 1 cm away from an outer shell of the blade surface is an outer shell.

5. The beautification machine for cutting hair according to any one of Claims 1 to 4, wherein
the skin information acquisition unit comprises a practitioner information input mechanism capable of inputting a state of skin of a person being treated.

6. The beautification machine for cutting hair according to any one of Claims 1 to 5, wherein
the main body comprises a skin information output unit that outputs the skin information acquired by the skin information acquisition unit to the treatment setting unit.

7. The beautification machine for cutting hair according to any one of Claims 1 to 6, wherein
the main body comprises a skin information transmission unit capable of transmitting the skin information acquired by the skin information acquisition unit to an outside.

8. The beautification machine for cutting hair according to any one of Claims 1 to 7, wherein
the main body comprises a threshold storage that stores the threshold, and
the threshold acquisition unit acquires the threshold stored in the threshold storage.

9. The beautification machine for cutting hair according to Claim 8, wherein
the threshold storage comprises a threshold updating unit capable of updating the threshold stored in the threshold storage based on threshold information acquired from an outside of the beautification machine for cutting hair.

10. The beautification machine for cutting hair according to any one of Claims 1 to 9, wherein
the main body comprises a threshold output unit that outputs the threshold acquired by the threshold acquisition unit to the treatment setting unit.

11. The beautification machine for cutting hair according to any one of Claims 1 to 10, wherein
the treatment setting unit comprises a basic function controller that controls a basic function of the treatment unit.

12. The beautification machine for cutting hair according to Claim 11, wherein
the treatment unit comprises an outer blade, and
the basic function controller comprises a contact pressure detection mechanism that detects a contact pressure of the outer blade to the skin, and a contact pressure changing unit capable of changing the contact pressure based on the contact pressure detected by the contact pressure detection mechanism.

13. The beautification machine for cutting hair according to Claim 12, wherein
the contact pressure changing unit comprises an outer blade position adjustment mechanism that adjusts a position of the outer blade.

14. The beautification machine for cutting hair according to Claim 12 or 13, wherein
the contact pressure changing unit comprises a notification unit that gives a notification of a contact state of the outer blade with the skin.

15. The beautification machine for cutting hair according to any one of Claims 1 to 14, wherein
the main body comprises a skin information transmission unit that transmits information to an information display unit capable of displaying the skin information.

16. The beautification machine for cutting hair according to Claim 15, wherein
the skin information transmission unit transmits information to an information display unit provided in the main body.

17. The beautification machine for cutting hair according to Claim 15 or 16, wherein
the skin information transmission unit transmits information to an information display unit provided in a terminal device.

18. A beautification system comprising:
the beautification machine for cutting hair according to any one of Claims 1 to 17; and
an information display unit that is provided independently of the beautification machine for cutting hair and is capable of displaying the skin information.

19. A beautification system comprising:
the beautification machine for cutting hair according to any one of Claims 1 to 17; and
a threshold transmission unit capable of transmitting data of the threshold acquired by the threshold acquisition unit.

20. A beautification system comprising:
the beautification machine for cutting hair according to any one of Claims 1 to 17; and
a skin information transmission unit capable of transmitting data of the skin information acquired by the skin information acquisition unit.
